# EUROPEAN PATENT APPLICATION

(11) **EP 1 053 748 A1**
(43) Date of publication of application: **22.11.2000**
(21) Application number: 99902840.0
(22) Date of filing: 05.02.1999
(51) Int. Cl.: A61K 31/557

(54) **REMEDIES FOR NEUROLOGICAL DISORDERS**

(30) Priority: 06.02.1998 JP 2570198
(71) Applicant: TEIJIN LIMITED, Osaka-shi Osaka 541-0054 (JP)
(72) Inventor: SUWA, Yorimasa, Teijin Limited, Hino-shi, Tokyo 191-0065 (JP); YOSHIOKA, Noboru, Teijin Limited, Hino-shi, Tokyo 191-0065 (JP); SUMI, Yoshihiko, Teijin Limited, Hino-shi, Tokyo 191-0065 (JP); ARAI, Takami, Teijin Limited, Hino-shi, Tokyo 191-0065 (JP); SAKURAI, Katsutoshi, Teijin Limited, Hino-shi, Tokyo 191-0065 (JP); NABESHIMA, Toshitaka, Nagoya Uty. School of Medc., Nagoya-shi, Aichi 466-0065 (JP); YAMADA, Kiyofumi, Nagoya Uty. School of Medicine, Nagoya-shi, Aichi 466-0065 (JP)
(74) Representative: Hallybone, Huw George
(86) International application number: JP9900502
(87) International publication number: WO9939714

(57) **Abstract**

It has been made clear that a specific prostaglandin such as isocarbacyclin derivatives is effective for the treatment of a disease caused by a nervous disorder or nervous disorder caused by an external wound. There are provided a drug for a nervous disorder comprising the above specific prostaglandin and a method for therapy of a nervous disorder.

## Description

### Field of the Invention

The present invention relates to a drug for a nervous disorder. More specifically, it relates to a drug for a nervous disorder which contains a prostaglandin having a specific structure as an active component.

### Prior Art

In a nervous system, each nerve cell can fulfill its function only when it extends its neurite (axon or dendrite) to form a network with other nerve cells through synapses or to interact with a muscle cell, chrotoplast or the like.

The survival of the nerve cell itself is maintained by exchanging transmitters and trophic factors with other nerve cells by interaction through the neurite.

Therefore, even when the nerve cell itself is not damaged fatally, if its neurite is damaged, its nerve function lowers and the nerve cell itself dies. Not only when the neurite is disconnected by an external wound or the like but also in the initial stage of many diseases derived from nervous disorders and at a portion around a site having a lesion, it is considered that a damage like above first occurs in the neurite.

Therefore, it is assumed that a drug having the function of repairing the damage in the neurite is extremely effective for many diseases caused by nervous disorders, nerve injuries caused by external wounds, and the like.

The diseases caused by nervous disorders include dementia of Alzheimer type, Parkinson disease, multiple sclerosis, lateral sclerosis, diabetic neuropathy and the like. Out of these, an increase in the number of cases of dementia of Alzheimer type is becoming a serious social problem to be solved with the arrival of the aged society.

Drugs which have been used for the treatment of dementia of Alzheimer type are roughly classified into a group of cerebral circulation improver and cerebral metabolism enhancer and a group of cholinergic drugs.

The cerebral circulation improver and cerebral metabolism enhancer are drugs for lesions in the blood vessels of the brain and sequelae thereof, which do not repair nervous disorders directly and have low effectiveness against dementia symptoms. Meanwhile, the cholinergic drugs have been developed based on pathological findings that cases of dementia of Alzheimer type have a marked lesion in the cholinergic nervous system and use thereof is authorized as the only drug for dementia of Alzheimer type. Since it has been made clear that in the pathology of the dementia of Alzheimer type, the cholinergic nervous system is not the only system which suffers a lesion, it is considered that the effectiveness of the cholinergic drugs is limited.

Under the situation, the development of a drug for dementia of Alzheimer type based on a novel made of action has been strongly desired. Especially, a drug having the function of repairing a lesion in the neurite as mentioned above is expected to show the highest effectiveness in the treatment of dementia of Alzheimer type if it is implemented.

Main drug evaluation systems which have been used for the search of drugs for dementia include in vitro systems such as a system for measuring the activity of inhibiting an enzyme that deradates a neurotransmitter, such as acetylcholinesterase, a system for measuring the activity of increasing the number of neurotransmitters using a slice of the brain, and a system for measuring the activity of promoting the outgrowth of a neurite or suppressing the death of a nerve cell using a dorsal root ganglion cell or PC12 cell and in vivo systems such as a model for causing a lesion specifically in a cholinergic system by administering scopolamine or destroying it electrically and a model for provoking a lesion by giving a low-oxygen stress with cerebral ischemia and carbon dioxide.

Most of these evaluation systems are rational as evaluation systems for cholinergic drugs and cerebral circulation improver and cerebral metabolism enhancer. However, they are unsatisfactory for the evaluation of new drugs for dementia because there is a great discrepancy between actual dementia, particularly dementia of Alzheimer type, and these experimental models, pathogenetically and pathologically.

Also in the system for measuring the activity of promoting the outgrowth of a neurite, it is considered that not only nerve cells which extend their neurites but also cells which interact with the extending neurites through synapses and glia cells exist around the nerve cells in vivo and that the outgrowth speeds and directions of the neurites are determined by various factors produced and released from these cells. Therefore, conventional systems are unsatisfactory for the evaluation of the activity of a compound which influences the outgrowth of the neurite of a nerve cell.

In the brain of a patient with the dementia of Alzheimer type, the loss of the nerve cells in the entorhinal cortex occurs as the most initial stage of the disease. Paying attention to this, the present inventors have succeeded in the regeneration of the axon of the nerve cell in the entorhinal cortex cut out and the reproduction of projection of a nerve onto the dentate gyrus of hippocampus as in the brain by cutting away the tissue of a portion containing both the entorhinal cortex and hippocampus from the brain of a young rat and culturing it in a 6-wells plate (European Journal of Neuroscience, vol. 6, pp. 1026-1037).

The major feature of dementia of Alzheimer type is that senile plaques are formed in the brain. There has been accumulated evidence which verifies that β-protein as the major component of the senile plaques which aggregates in the brain to form amyloid and deposits on the tissue of the brain to exhibit neurotoxicity is the major cause of dementia of Alzheimer type. Paying attention to this fact, the present inventors succeeded in the formation of a model whose learning and memory are impaired by burying an osmotic pressure pump under the skin of a back portion of a rat to infuse β-protein into the ventricle of the brain continuously (Neuroscience Letters, vol. 170, pp. 63-66, 1994).

The above system for the outgrowth and regeneration of a neurite using the culture of a brain tissue and the above model obtained by infusing β-protein into the ventricle of the brain continuously are extremely rational as systems for evaluating drugs for dementia of Alzheimer type from pathological and pathogenetical points of view. At the same time, they are the most suitable for the evaluation of the activity of a drug for general diseases caused by nervous disorders or nerve injuries caused by external wounds.

Meanwhile, it has been made clear that prostaglandins have various activities such as the suppression of platelet aggregates, angiectatic pressure reduction, the suppression of secretion of acid in the stomach, the shrinkage of smooth muscle, the protection of cells and the diuresis. Natural prostaglandins and derivatives thereof which are given chemical and biological stabilities have been developed as medicines for use in the fields of obstetrics and gynecology, digestive organ, circulatory organ and ophthamology.

In the field of the brain and nerve, no prostaglandins have been developed as medicines but it has been reported that some types of prostaglandins exhibit the function of protecting the nerve cells of the brain (Neuroscience Letters, vol. 160, pp. 106, 1993 and JP-A 8-245498) (the term "JP-A" as used herein means an "unexamined published Japanese patent application").

However, it has been unknown that prostaglandins have the function of repairing a damage in a neurite and a potential as a drug for dementia of Alzheimer type.

### Summary of the Invention

It is an object of the present invention to provide a drug for a disease caused by a nervous disorder or a nerve injury caused by an external wound. Examples of the disease caused by a nervous disorder include neurodegenerative diseases accompanied by dementia such as dementia of Alzheimer type, Parkinson disease, multiple sclerosis, lateral sclerosis and diabetic neuropathy, or motor disorders, and the functional disorders of the brain caused by cerebral infarction and cerebral apoplexy.

The present inventors have repeated intensive studies to attain the above objects, have developed a new method for measuring the degree of promoting the outgrowth of a neurite using a cerebral tissue culture system and have found that the above object can be attained by a prostaglandin having a specific structure using the above measurement method and an animal model of dementia of Alzheimer type injected with β-protein into the ventricle of the brain continuously as an evaluation system. The present invention has been accomplished by the above finding.

That is, the present invention is a drug for a nervous disorder which contains a prostaglandin represented by the following formula (I): 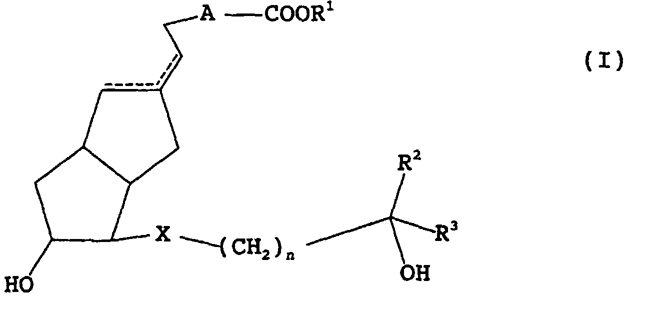 wherein R¹ is a hydrogen atom, C₁∼C₁₀ alkyl group or 1-equivalent cation, R² is a hydrogen atom, methyl group or vinyl group, R³ is a linear or branched C₃∼C₁₀ alkyl group, linear or branched C₃∼C₁₀ alkenyl group, linear or branched C₃∼C₁₀ alkynyl group, C₃∼C₁₀ cycloalkyl group which may be substituted, phenyl group which may be substituted, phenoxy group which may be substituted, or linear or branched C₁∼C₆ alkyl group which is substituted with a phenyl group which may be substituted, phenoxy group which may be substituted or C₃∼C₁₀ cycloalkyl group which may be substituted, n is 0 or 1, A is -CH₂-CH₂-, -CH=CH- or -O-CH₂-, X is -CH₂-CH₂-, -CH(NH₂)-CH₂-, -CH=CH-, -C(NH₂)=CH- or -C≡C-, and the bond 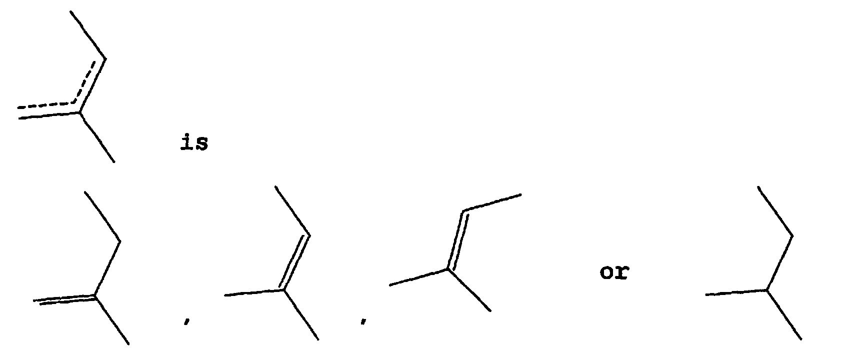 with the proviso that when A is -O-CH₂- and X is -CH(NH₂)-CH₂-or -CH(NH₂)=CH-, the bonding direction may be opposite, the following formula (III): 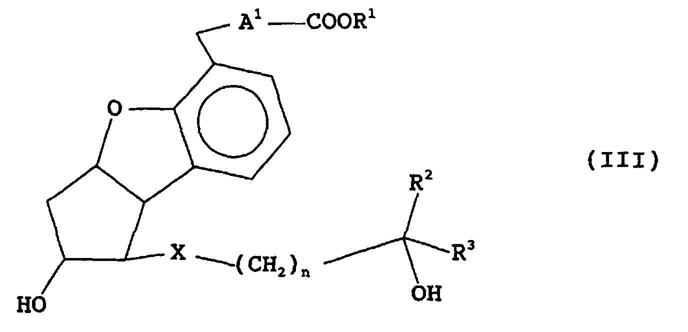 wherein R¹, R², R³, X and a are as defined in the above formula (I), and A¹ is -CH₂-CH₂- or -CH=CH-, with the proviso that when X is -CH(NH₂)-CH₂- or -C(NH₂)=CH-, the bonding direction may be opposite,
or the following formula (IV): 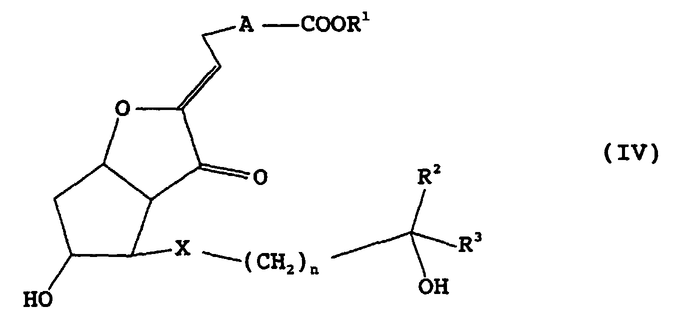 wherein R¹, R², R³, A, X and n are as defined in the formula (I) with the same proviso as in the formula (I), and/or an optical isomer thereof as an active ingredient(s).

### Brief Description of the Drawings

Fig. 1 is a schematic diagram of an example of a horizontally cut slice of the brain of an SD rat; and
Fig. 2 is a diagram for explaining the preparation of a culture sample for the evaluation of the activity of promoting the outgrowth of a neurite using a horizontally cut slice of the brain of an SD rat.

### Detailed Description of the Preferred Embodiment

In the above formula (I), the above formula (III) and the above formula (IV), R¹ is a hydrogen atom, C₁∼C₁₀ alkyl group or 1-equivalent cation.

Examples of the C₁∼C₁₀ alkyl group include methyl, ethyl, n-propyl, iso-propyl, n-butyl, sec-butyl, tert-butyl, n-pentyl, n-hexyl, n-heptyl, n-octyl, n-nonyl and n-decyl groups. C₁∼C₃ alkyl groups are preferred and a methyl group is particularly preferred.

Preferred examples of the 1-equivalent cation include sodium, potassium, lithium, rubidium, cesium, 1/2 calcium, 1/2 magnesium and 1/3 aluminum. Lithium and sodium are particularly preferred.

R² is a hydrogen atom, methyl group or vinyl group. When n = 0, a hydrogen atom is preferred and when n = 1, a methyl group is preferred.

R³ is a linear or branched C₃∼C₁₀ alkyl group, linear or branched C₃∼C₁₀ alkenyl group, linear or branched C₃∼C₁₀ alkynyl group, C₃∼C₁₀ cycloalkyl group which may be substituted, phenyl group which may be substituted, phenoxy group which may be substituted, or linear or branched C₁∼C₆ alkyl group which is substituted with a phenyl group which may be substituted, phenoxy group which may be substituted or C₃∼C₁₀ cycloalkyl group which may be substituted.

Examples of the linear or branched C₃∼C₁₀ alkyl group include n-propyl, n-butyl, n-pentyl, n-hexyl, n-heptyl, n-octyl, n-decyl, 1-methylpentyl, 1-methylhexyl, 1,1-dimethylpentyl, 2-methylpentyl, 2-methylhexyl, 5-methylhexyl and 2,5-dimethylhexyl groups. Out of these, n-butyl, n-pentyl, n-hexyl, (R)-, (S)- or (RS)-1-methylpentyl, and (R)-, (S)- or (RS)-2-methylhexyl groups are preferred. C₃∼C₆ alkyl groups are more preferred, out of which butyl and pentyl groups are particularly preferred.

Examples of the linear or branched C₃∼C₁₀ alkenyl group include 2-butenyl, 2-pentenyl, 3-pentenyl, 2-hexenyl, 4-hexenyl, 2-methyl-4-hexenyl and 2,6-dimethyl-5-heptenyl groups.

Examples of the linear or branched C₃∼C₁₀ alkynyl group include 2-butynyl, 2-pentynyl, 3-pentynyl, 2-hexynyl, 3-hexynyl, 4-hexynyl, 2-octynyl, 5-decynyl, 1-methyl-3-pentynyl, 1-methyl-3-hexynyl and 2-methyl-4-hexynyl groups. C₅∼C₇ alkynyl groups are preferred, out of which 3-pentynyl, 1-methyl-3-pentynyl, 3-hexynyl and 1-methyl-3-hexynyl groups are particularly preferred.

A cycloalkyl group moiety of the C₃∼C₁₀ cycloalkyl group which may be substituted is cyclopropyl, cyclobutyl, cyclopentyl, cyclohexyl, cycloheptyl or cyclooctyl group. The substituent of the cycloalkyl group is a C₁∼C₆ alkyl group or halogen atom. The number of substituents may be plural.

Examples of the C₃∼C₁₀ cycloalkyl group which may be substituted include cyclopropyl, cyclobutyl, cyclopentyl, cyclohexyl, cycloheptyl, cyclooctyl, (C₁∼C₆)alkylcyclopentyl, (C₁∼C₄)alkylcyclohexyl, dimethylcyclopentyl, dimethylcyclohexyl, chlorocyclopentyl, bromocyclohexyl, iodocyclopentyl and fluorocyclohexyl groups. C₅∼C₆ cycloalkyl groups are preferred, out of which cyclopentyl and cyclohexyl groups are particularly preferred.

The substituent of the phenoxy group which may be substituted or phenoxy group which may be substituted is a halogen atom, hydroxyl group, C₂∼C₇ acyloxy group, C₁∼C₄ alkyl group which may be substituted with a halogen atom, C₁∼C₄ alkoxyl group which may be substituted with a halogen atom, nitrile group, carboxyl group or (C₁∼C₆)alkoxycarbonyl group. The halogen atom is preferably a fluorine, chlorine or bromine atom. A fluorine or chlorine atom is particularly preferred. Examples of the C₂∼C₇ acyloxy group include acetoxy, propionyloxy, n-butyryloxy, iso-butyryloxy, n-valeryloxy, iso-valeryloxy, caproyloxy, enanthyloxy or benzoyloxy group. Further, preferred examples of the C₁∼C₄ alkyl group which may be substituted with a halogen atom include methyl, ethyl, n-propyl, iso-propyl, n-butyl, chloromethyl, dichloromethyl and trifluoromethyl groups. Preferred examples of the C₁∼C₄ alkoxy group which may be substituted with a halogen atom include methoxy, ethoxy, n-propoxy, iso-propoxy, n-butoxy, chloromethoxy, dichloromethoxy and trifluoromethoxy groups. Examples of the (C₁∼C₆)alkoxycarbonyl group include methoxycarbonyl, ethoxycarbonyl, butoxycarbonyl and hexyloxycarbonyl groups.

The substituted phenyl group or substituted phenoxy group can have one to three, preferably one of the above substituents.

In the linear or branched C₁∼C₆ alkyl group substituted with a phenyl group which may be substituted, phenoxy group which may be substituted or C₃∼C₁₀ cycloalkyl group which may be substituted, preferred examples of the phenyl group which may be substituted and phenoxy group which may be substituted are the same as those enumerated above.

Preferred examples of the C₃∼C₁₀ cycloalkyl group are the same as those enumerated above. Examples of the substituent are also the same as the above substituents of the substituted phenyl group or substituted phenoxy group. Examples of the linear or branched C₁∼C₆ alkyl group include methyl, ethyl, propyl, iso-propyl, butyl, iso-butyl, sec-butyl, t-butyl, pentyl and hexyl groups. The substituent may be bonded at any position of the alkyl group.

Out of the prostaglandins represented by the above formula (I) and/or optical isomers thereof, a prostaglandin represented by the following formula (II): 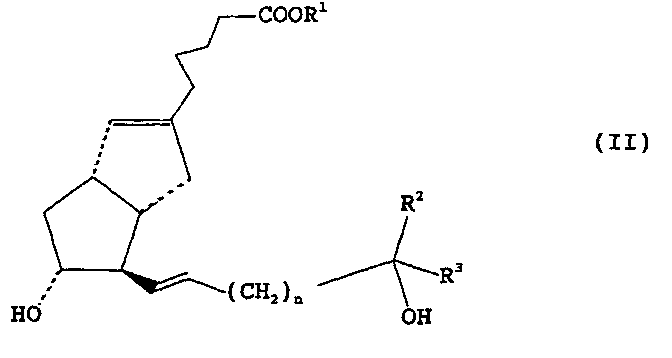 wherein R¹, R², R³ and n are as defined in the above formula (I),
and/or an optical isomer thereof are/is preferred.

When A is -O-CH₂- in the above formulas (I) and (IV), the methylene group (-CH₂-) thereof is preferably bonded to the carboxyl group (-COOR¹). When X is -CH(NH₂)-CH₂- or - C(NH₂)=CH- in the above formulas (I), (III) and (IV), the carbon atom bonded to the amino group is preferably linked to the cyclopentane ring.

Illustrative examples of the prostaglandin used as an active component in the present invention are given below.
(1) 9(O)-methano-Δ^{6(9α)}-prostaglandin I₁ (isocarbacycline derivatives)
(2) 20-methyl-9(O)-methano-Δ^{6(9α)}-prostaglandin I₁
(3) 16-methyl-9(O)-methano-Δ^{6(9α)}-prostaglandin I₁
(4) 16,16-dimethyl-9(O)-methano-Δ^{6(9α)}-prostaglandin I₁
(5) 17-methyl-9(O)-methano-Δ^{6(9α)}-prostaglandin I₁
(6) 17,20-dimethyl-9(O)-methano-Δ^{6(9α)}-prostaglandin I₁
(7) (17R)-configuration of (6)
(8) (17S)-configuration of (6)
(9) 15-methyl-9(O)-methano-Δ^{6(9α)}-prostaglandin I₁
(10) 17,18-dehydro-9(O)-methano-Δ^{6(9α)}-prostaglandin I₁
(11) 20-isopropylidene-17-methyl-9(O)-methano-Δ^{6(9α)}-prostaglandin I₁
(12) 18,18,19,19-tetradehydro-16-methyl-9(O)-methano-Δ^{6(9α)}-prostaglandin I₁
(13) 18,19-dehydo-16-methyl-9(O)-methano-Δ^{6(9α)}-prostaglandin I₁
(14) (16S)-configurations of (3), (12) and (13)
(15) (16R)-configurations of (3), (12) and (13)
(16) 16,17,18,19,20-pentanol-15-cyclopentyl-9(O)-methano-Δ^{6(9α)}-prostaglandin I₁
(17) 16,17,18,19,20-pentanol-15-cyclohexyl-9(O)-methano-Δ^{6(9α)}-prostaglandin I₁
(18) 17,18,19,20-tetranol-16-(p-fluorophenoxy)-9(O)-methano-Δ^{6(9α)}-prostaglandin I₁
(19) 17,18,19,20-tetranol-16-cyclohesyl-9(O)-methano-Δ^{6(9α)}-prostaglandin I₁
(20) 15-deoxy-16-hydroxy-9(O)-methano-Δ^{6(9α)}-prostaglandin I₁
(21) 15-deoxy-16-hydroxy-16-methyl-9(O)-methano-Δ^{6(9α)}-prostaglandin I₁
(22) (16S)-configurations of (20) and (21)
(23) (16R)-configurations of (20) and (21)
(24) methyl esters of (1) to (23)
(25) ethyl esters of (1) to (23)
(26) butyl esters of (1) to (23)
(27) sodium salts of (1) to (23)
(28) potassium salts of (1) to (23)
(29) ammonium salts of (1) to (23)
(30) 8-, 9-, 11-, 12- and 15-position stereoisomers of compounds (1) to (29)
(31) 17,18,19,20-tetranol-16-(m-tolyl)-9(O)-methano-Δ^{6(9α)}-prostaglandin I₁
(32) 17,18, 19,20-tetranol-16-(o-tolyl)-9(O)-methano-Δ^{6(9α)}-prostaglandin I₁
(33) 17,18,19,20-tetranol-16-(p-tolyl)-9(O)-methano-Δ^{6(9α)}-prostaglandin I₁
(34) (15S)-configurations of (31) to (33)
(35) (15R)-configurations of (31) to (33)
(36) methyl esters of (31) to (35)
(37) ethyl esters of (31) to (35)
(38) butyl esters of (31) to (35)
(39) sodium salts of (31) to (35)
(40) potassium salts of (31) to (35)
(41) ammonium salts of (31) to (35)
(42) (5E)-(16RS)-16-methyl-9(O)-methano-prostaglandin I₂
(43) (5E)-(16S)-16,20-dimethyl-3-oxa-13,14,18,18,19,19-hexadehydro-9(O)-methano-prostaglandin I₂
(44) (5E)-(16R)-16,20-dimethyl-3-oxa-13,14,18,18,19,19-hexadehydro-9(O)-methano-prostaglandin I₂
(45) (5E)-(16RS)-16,20-dimethyl-3-oxa-13,14,18,18,19,19-hexadehydro-9(O)-methano-prostaglandin I₂
(46) (5E)-(16S)-16,20-dimethyl-3-oxa-13,14,18,18,19,19-hexadehydro-9(O)-methano-prostaglandin I₂-tris-(hydroxymethyl)-aminomethane salt
(47) 16-methyl-18,18,19,19-tetradehydro-5,6,7-trinol-4,8-inter-m-phenylene-prostaglandin I₂
(48) 16-methyl-18,18,19,19-tetradehydro-5,6-7-trinol-4,8-inter-m-phenylene-prostaglandin I₂-methyl ester
(49) 18,18,19,19-tetradehydro-5,6,7-trinol-4,8-inter-m-phenylene-prostaglandin I₂
(50) 18,18,19,19-tetradehydro-5,6,7-trinol-4,8-inter-m-phenylene-prostaglandin I₂-methyl ester
(51) 20-methyl-18,18,19,19-tetradehydro-5,6,7-trinol-4,8-inter-m-phenylene-prostaglandin I₂
(52) 20-methyl-18,18,19,19-tetradehydro-5,6,7-trinol-4,8-inter-m-phenylene-prostaglandin I₂-methyl ester
(53) 16,20-dimethyl-18,18,19,19-tetradehydro-5,6,7-trinol-4,8-inter-m-phenylene-prostaglandin I₂
(54) 16,20-dimethyl-18,18,19,19-tetradehydro-5,6,7-trinol-4,8-inter-m-phenylene-prostaglandin I₂-methyl ester
(55) 20-ethyl-16-methyl-18,18,19,19-tetradehydro-5,6,7-trinol-4,8-inter-m-phenylene-prostaglandin I₂
(56) 20-ethyl-16-methyl-18,18,19,19-tetradehydro-5,6,7-trinol-4,8-inter-m-phenylene-prostaglandin I₂-methyl ester
(57) 16-methyl-2,3,18,18,19,19-hexadehydro-5,6,7-trinol-4,8-inter-m-phenylene-prostaglandin I₂
(58) 16-methyl-2,3,18,18,19,19-hexadehydro-5,6,7-trinol-4,8-inter-m-phenylene-prostaglandin I₂-methyl ester
(59) 2,3,18,18,19,19-hexadehydro-5,6,7-trinol-4,8-inter-m-phenylene-prostaglandin I₂
(60) 16-methyl-13,14,18,18,19,19-hexadehydro-5,6,7-trinol-4,8-inter-m-phenylene-prostaglandin I₂
(61) 16-methyl-13,14,18,18,19,19-hexadehydro-5,6,7-trinol-4,8-inter-m-phenylene-prostaglandin I₂-methyl ester
(62) 20-methyl-19,19,20,20-tetradehydro-5,6,7-triniol-4,8-inter-m-phenylene-prostaglandin I₂
(63) 17,17,18,18-tetradehydro-5,6,7-trinol-4,8-inter-m-phenylene-prostaglandin I₂
(64) 7-oxo-prostaglandin I₂
(65) mixture of (64) and [R-(R*,S*)]-α-[1-(methylamino)-ethyl]benzene methanol (1:1)

The optical isomers of the above compounds and mixtures of the above compounds and optical isomers thereof in any ratio may be used in the present invention.

A description is subsequently given of a method for evaluating the activity of promoting the outgrowth of a neurite, which was newly developed by the present inventors in the present invention.

This method is a physiological activity evaluation method using the promotion of outgrowth of a neurite as an index when a tissue slice of the brain or nerve is cultured. It is characterized in that the tissue of the brain or nerve of a mammal after its birth is used as a material, a portion where a culture medium is existent and a portion where a tissue slice is existent are separated from each other spatially, the tissue slice is cultured on the surface of a support which permits the permeation of the culture medium into the tissue slice, and the support is an area which allows for the outgrowth of a neurite and is to be observed.

The expression "tissue slice of the brain or nerve" means part of the brain and nervous system extracted from a living animal and includes a nerve cell which can extend its neurite. As for the size thereof, it is preferably a slice having a maximum diameter of 2 cm or less and a thickness of 0.5 mm or less, preferably a maximum diameter of 2 mm or less.

This evaluation method is to measure the length of the neurite extending from the soma of a nerve cell existent during culture or a parameter reflecting it. The method for measuring it is to visualize a neurite out of a structure contained in the tissue slice of the brain or nerve. To this end, a method using an optical/imaging apparatus including a microscope, or a method using an optical/imaging apparatus after a culture tissue slice is dyed may be used.

Methods for dying a tissue include a method using a labeling compound which is transferred along a neurite, such as biocytin, biotin dextran, PHA-L (Phaseolus vulgaris-leucoagglutinin) or DiI (1,1'-dioctadecyl-3,3,3',3'-tetramethylindocarbocyanine perchlorate) and a method using an antibody such as an anti-neurofilament antibody.

The expression "mammal" generally means a laboratory animal, preferably a rodent, particularly preferably a rat. The expression "mammel after its birth" means a 7 days or more old animal.

Further, the expression "support" means what is designed to separate a liquid culture medium from a tissue slice to be cultured spatially and to enable active ingredients contained in the culture medium to permeate into the tissue slice. It is, for example, a gel collagen layer having an appropriate thickness. The collagen layer may be adhered to a film having holes of an appropriate size which can support the collagen layer, such as a polycarbonate membrane filter. That is, the under surface of the film to which the collagen layer is adhered is immersed in the culture medium and a tissue slice is placed on the top surface of the gel collagen layer to be cultured.

The evaluation system is constructed by carrying out the co-culture of a tissue slice containing entorhinal cells and a tissue slice containing hippocampal cells. The distance between the both tissue slices is preferably 0.1 mm to 1 mm. The method of preparing these tissue slices in this case is to slice the brain and cut out a required portion from the slice.

The above culture of the tissue slice of the brain or nerve is carried out without a serum. The culture medium is prepared by reducing the amounts of glutamic acid and aspartic acid or removing them from a DMEM:F12 (1:1) medium and preferably adding transferrin, insulin, hydrocortisone, sodium selenide, progesterone and glutamine. The concentrations of the glutamic acid, aspartic acid, transferrin, insulin, hydrocortisone, sodium selenide, progesterone and glutamine are less than 7 mg/l, less than 6.5 mg/l, 10 to 200 mg/l, 1 to 20 mg/l, 2 to 100 nM, 2 to 100 nM, 2 to 100 nM and 1 to 20 mM, respectively.

What the drug for nervous disorder of the present invention is used for is not particularly limited. The drug of the present invention is particularly useful for mammals and advantageously used for domestic animals, laboratory animals, pets and human beings.

The drug for nervous disorder of the present invention can be used to treat the disease of an animal or human being. The disease is not particularly limited and may be caused by a nervous disorder. Examples of the disease include the degenerative diseases of the brain or nervous system, more specifically neurodegenerative diseases of the brain or nervous system accompanied by dementia such as dementia of Alzheimer type, Parkinson disease, multiple sclerosis, lateral sclerosis and diabetic neuropathy, or motor disorders, the functional disorders of the brain caused by cerebral infarction and cerebral apoplexy, and nerve injuries caused by external wounds.

The administration of the drug of the present invention is not particularly limited but it is preferably oral, percutaneous or pernasal administration, intravenous injection, or administration into the abdominal cavity, rectum or cerebral ventricle.

To apply the prostaglandin of the present invention, salt thereof or clathrate compound thereof clinically, it is preferred that a medical composition comprising the prostaglandin as an active ingredient and a solid or liquid carrier which is permitted pharmaceutically should be first prepared and preparations should be obtained by adding diluents, that is, additives such as an excipient, and stabilizer to the composition as required. Preparations to be injected of the prostaglandin of the present invention which should be administered therapeutically must be generally in a sterile state. Sterility can be easily attained by filtering the preparations with a sterilized membrane filter such as a membrane filter having a pore diameter of 0.2 µm.

The ratio of the above active ingredient to the carrier component in the medical composition can be changed between 0.01 and 90 %W/W. The therapeutically effective dosage of the composition, which depends on administration, age and type of disease, may be 0.01 µg to 1,000 mg/day/person, preferably 0.01 µg to 10 mg/day/person. It is desired to determine the absorption efficiency into the body of each compound through each administration route independently by a pharmaceutically known method.

As for the form and administration of each preparation, it may be administered orally in a granular, fine particle, powder, pill, tablet, capsule or liquid form, or non-orally as a local preparation such as a suppository, aerosol, ointment or skin patch. It may be injected into the vein, artery or muscle or under the skin. It may be powdery for injection and prepared before use. Further, it may be administered pernasally or into the abdominal cavity, rectum or cerebral ventricle.

An organic or inorganic, solid or liquid carrier or diluent for medical use which is suitable for oral, rectal or non-oral administration may be used to prepare the prostaglandin of the present invention as medical preparations.

Typical examples of the carrier or diluent which can be incorporated into a tablet or capsule include a binder such as an acacia, corn starch or gelatin, excipient such as fine crystalline cellulose, disintegrant such as corn starch or alginic acid, lubricant such as magnesium stearate and sweetener such as cane sugar or alginic acid. When the preparation is in the form of a capsule, a liquid carrier such as fatty oil may be contained in addition to the above materials. Other various materials may be used as coatings or modifiers for improving the physical shape of an administration unit. Preparations can be obtained from a sterilized composition for injection in accordance with conventional pharmaceutical methods. For example, it is preferred to dissolve or suspend an active compound in an excipient such as water or natural vegetable oil, or a synthetic aliphatic excipient such as ethyl oleate. A buffering agent such as a citrate, acetate or phosphate, or an antioxidant such as ascorbic acid may be added in accordance with allowed pharmaceutical methods.

An excipient such as lactose, starch or crystalline cellulose; a binder such as carboxymethyl cellulose, methyl cellulose or polyvinyl pyrrolidone; and a disintegrant such as sodium alginate, sodium hydrogen carbonate or sodium lauryl sulfate are used to form a tablet preparation in accordance with generally used methods.

The above excipient and the like are used to form pill, powder and granular preparations in accordance with generally used methods as well. A glycerin ester such as tricaprylin or triacetin, or an alcohol such as ethanol is used to form liquid and suspension preparations in accordance with generally used methods. A granular, powder or liquid preparation is filled into a capsule such as a gelatin capsule to form a capsule preparation.

The prostaglandin of the present invention may be converted into a cyclodextrin clathrate compound as a preparation to be orally administered. The clathrate compound is prepared by adding a solution of cyclodextrin dissolved in water and/or an organic solvent which is easily mixed with water to a solution of a prostaglandin dissolved in an organic solvent which is easily mixed with water. After the mixture is heated, the cyclodextrin clathrate compound of interest is isolated by separating the product by vacuum concentration, filtration with a filter under cooling, or decantation. The ratio of the organic solvent to water changes according to the solubilities of the starting materials and the product. The temperature during the preparation of the cyclodextrin clathrate compound is preferably not higher than 70°C. α-, β- or γ-cyclodextrin or a mixture thereof may be used to prepare the cyclodextrin clathrate compound. The prostaglandin can experience an increase in stability when it is converted into a cyclodextrin clathrate compound.

Preparations to be administered hypodermically, intramuscularly or intravenously are injections in the form of an aqueous or non-aqueous solution. The aqueous solution injections include physiologic saline or the like. The non-aqueous solution injections are composed of propylene glycol, polyethylene glycol, olive oil or ethyl oleate and optionally an antiseptic or a stabilizer is added thereto. The injections are made aseptic by filtration with a membrane filter, blending a fungicide, or the like.

The preparations to be administered hypodermically include ointments and creams. The ointments are prepared by using oils such as castor oil and olive oil; or vaseline and the creams are prepared by using fatty oil; or an emulsifier such as diethylene glycol or sorbitan monofatty acid ester in accordance with generally used methods.

For rectal administration, general suppositories such as a gelatin soft capsule are used.

Preparations for non-oral administration can be administered as emulsions. That is, a fatty emulsion prepared by adding water to a uniform solution of vegetable oil such as soybean oil, phospholipid such as lecithin and isocarbacyclin derivatives of the present invention and homogenizing the resulting mixture with a homogenizer such as a pressure injection homogenizer or ultrasonic homogenizer may be used as an injection.

As is obvious from the above description, it can be understood that according to the present invention, there are also provided use of a prostaglandin represented by the above formula (I), (III) or (IV) and/or an optical isomer thereof as an active component(s) for the treatment of a nervous disorder as well as therapy of a nervous disorder characterized by administering the prostaglandin represented by the above formula (I), (III) or (IV) and/or an optical isomer thereof in a dose effective for the treatment of the nervous disorder to a patient having the nervous disorder.

### Examples

The following examples are given to further illustrate the present invention.

### Reference Example 1

### method of evaluating activity of promoting the outgrowth of neurite (1)

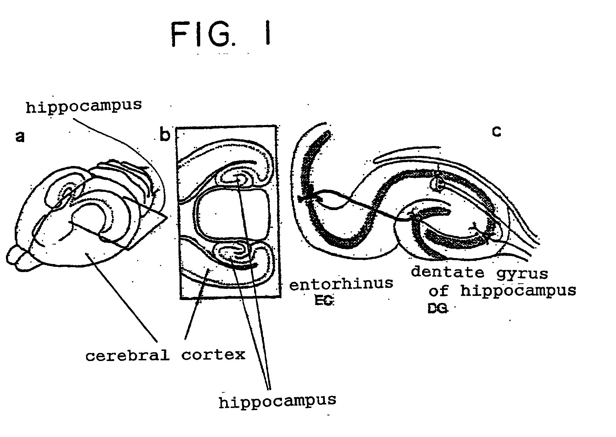

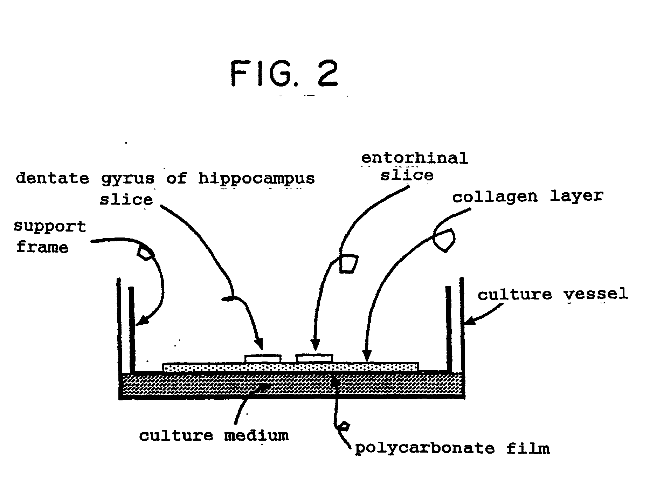

The brain of a 7-day old SD rat is sliced horizontally to obtain a plane containing both entorhinus and hippocampus (to be referred to as "horizontally cut slice" hereinafter; see Fig. 1; In Fig. 1, "a" is a diagram of the hemisphere of the cerebrum and therearound of the rat, "b" shows part of the horizontally cut slice and "c" shows a portion including both entorhinus and hippocampus; a nerve cell extending its neurite from the entorhinal cortex to the dentate gyrus of hippocampus is illustrated). A slice piece for the entorhinal cortex and a slice piece for the dentate gyrus of hippocampus are prepared from this horizontally cut slice having a thickness of 325 µm. The slice of the dentate gyrus of hippocampus and the slice of the entorhinal cortex are cultured on a gel collagen layer using a 5 % FCS culture medium in such a manner that the distance between the slices becomes approximately 500 µm. The collagen layer is prepared by coating a collagen gel on a polyethylene terephthalate porous film stretched on a commercially available support frame (see Fig. 2).

24 hours after the start of culture, the culture medium is replaced by a serum-free culture medium containing a material to be tested and further cultured for 1 day or 4 days. Thereafter, crystalline powders of biocytin are placed on the slice of the entorhinal cortex and cultured for another day (the neurites of the nerve cells contained in the slice grow into the collagen layer by this culture. The added biocytin is taken into the nerve cell of the entorhinal cortex and carried in a forward direction in the neurite so that it was existent in the entire neurite).

The cultured slice is fixed with paraformaldehyde and the neurite (a portion where biocytin is existent) is dyed with labeled avidin.

A photo of the dyed sample is taken, a region occupied by the neurite extending into the collagen layer from the slice of the entorhinal cortex is outlined on the photo, and the area of this outlined portion is calculated to measure the extension rate of the neurite.

### Reference Example 2

### method of evaluating activity of promoting the outgrowth of neurite (2)

The preparation, culture and dyeing after culture of a slice are carried out in the same manner as in Reference Example 1.

A photo of the dyed sample is taken and the number of neurites passing through a line segment having a fixed length (750 µm) at a fixed distance (200 µm) from the slice of the entorhinal cortex in the collagen layer is counted to measure the outgrowth rate of the neurite.

### Reference Example 3

### method of measuring short-term working memory using Morris water maze

This is carried out in conformity with the method of Morris (Journal of Neuroscience Methods, Vol. 11, pp.47-60, 1984).

A transparent acrylic platform (a diameter of 10 cm and a height of 25 cm) and a blue vinyl chloride round pool (a diameter of 140 cm and a height of 45 cm) filled with water to a height of 27 cm so that the platform is hidden under the water are used. The pool is divided into four quadrants, the platform is installed at the center of the fourth quadrant (35 cm from the center of the pool), and lights and drawing papers are placed around the pool to give spatial clues. A rat is thrown into the pool irregularly from 5 positions (the centers of the first, second and third quadrants and the boundaries between the first and second quadrants and between the second and third quadrants) by directing the head of the rat toward the wall of the pool as a memory acquisition trial. When it arrives at the platform and stays there for 15 seconds, it is considered that it recognizes the platform and when it swims again within, it is considered that it does not recognize the platform and the measurement is continued. The maximum observation time for one trial is 90 seconds, two trials are made per day, and the measurement is carried out for 5 consecutive days (10 trials in total).

A working memory test is carried out for 3 days after the above trials and the time elapsed from the time when a rat is thrown into the water to the time when it arrives at the platform (escape latency) is measured using a video camera set above the pool. On the first day of the working memory test, the position of the platform is moved by 180°C in a left direction from the position of learning and training (second quadrant). Like the memory acquisition trial, a rat is thrown into the pool by directing its head toward the wall of the pool to measure its escape latency. When it arrives at the platform and stays there for 15 seconds, it is returned to a home cage (1st trial). After 1 minute, the rat is thrown into the pool again to measure its escape latency and caused to stay on the platform for 15 seconds and returned to the home cage. This is repeated 4 times (2nd to 5th trials). The number of positions from which the rat is thrown into the pool is 5: the centers of the first, third and fourth quadrants and the boundaries between the first and fourth quadrants and between the third and fourth quadrants. The platform is moved by 90° from the position of learning and training (first quadrant) on the second day of the working memory test, and by 270°(third quadrant) on the third day to measure its escape latency in the same manner as described above. The maximum observation time of each trial is 90 seconds. Five trials are made each day for 3 days (15 trials in total) and the mean value of escape latency of second to fifth trials is taken as an index for the working memory.

### Reference Example 4

### method of measuring learning and memory abilities of rat by step- through passive avoidance test

A step-through passive avoidance test apparatus which consists of two rooms defined by a guillotine door is used as an experimental apparatus. That is, one room is a bright room (floor size of 15 cm x 25 cm, height of 15 cm) made of a transparent acrylic board and the other is a dark room (same in size) made of a black acrylic board. A stainless steel grid having a diameter of 4 mm is installed on the floor of the dark room at intervals of 15 mm and connected to an apparatus for applying an electric shock (shock generator scrambler).

A rat is caused to freely search the apparatus by opening the guillotine door for 1 minute, the door is closed, the rat is placed in the bright room as an acquisition trial, and the door is opened after 30 seconds. After the four feet of the rat enter the dark room, the door is closed and an electric shock is applied immediately. The strength of the electric shock is set to 0.5 mA x 5 seconds. Thereafter, the rat is placed in the bright room immediately and training is repeated until the rat stays in the bright room for 120 seconds even when the guillotine door is opened in the same procedure. After 24 hours of the acquisition trial, the rat is placed in the bright room as a retention trial, and the time elapsed from the time when the guillotine door is opened after 30 seconds to the time when the four legs of the rat enter the dark room is measured (step-through latency). The maximum observation time for the retention trial is set to 300 seconds.

### Reference Example 5

### Preparation of animal model of dementia of Alzheimer type by injection of β-amyroid protein into cerebral ventricle continuously

7 week old Wistar male rats (weight of 220 to 250 g) are used (N = 5-10).

β-amyloid protein (1-40) or β-amyloid protein (1-42) is dissolved in a 35% acetonitrile/0.1 % TFA, the resulting solution is injected into a mini osmotic pump (volume of 230 µl, 0.5 µl/hour) to 300 pmol/day, and the pump is connected to a dental injection needle by a polyethylene tube. A group of controls are connected to a pump filled with β-amyloid protein (40-1). The rats are anesthetized with pentobarbital (50 mg/kg, i.p.), the head skins thereof are cut open, the skulls thereof are drilled with a microdrill according to the Atlas of the rat brain, an injection needle is inserted into the hole so that the end of the needle is situated within the lateral ventricle of the brain (A = -0.3 mm, L = 1.2 mm, H = 4.5 mm), and the needle is fixed by dental cement. The osmotic pressure pump is buried under the skin of a back portion.

A water-maze test is carried out in the same manner as in Reference Example 3 nine days after the operation of burying the mini osmotic pump, or a passive avoidance test is carried out in the same manner as in Reference Example 4 thirteen or fourteen days after the operation to check if a group of rats dosed with β-amyloid protein (1-40) or β-amyloid protein (1-42) have lower learning and memory abilities than a group of rats dosed with β-amyloid protein (40-1).

### Example 1

### evaluation of activity of promoting the outgrowth of neurite (1)

### compound to be tested: (16S)-15-deoxy-16-hydroxy-16-methyl-9(O)-methano-Δ^{6(9α)}-prostaglandin I₁

The activity of promoting the outgrowth of a neurite was measured with the evaluation method of Reference Example 1.

In Experiments 1, 2 and 3, the material to be tested was added and cultured for 1 day, and then biocytin was added. In Experiment 4, the material to be tested was added and cultured for 4 days, and then biocytin was added. The results are shown below.

### 〈Experiment 1〉

| | mean value ± standard error (unit: mm²) |
|---|---|
| no addition of compound to be tested | 0.37 ± 0.25 (n=6) |
| 0.01 µM of compound to be tested | 1.47 ± 0.60 (n=5) |

### 〈Experiment 2〉

| | mean value ± standard error (unit: mm²) |
|---|---|
| no addition of compound to be tested | 0.81 ± 0.11 (n=9) |
| 0.01 µM of compound to be tested | 1.24 ± 0.16 (n=9) |

### 〈Experiment 3〉

| | mean value ± standard error (unit: mm²) |
|---|---|
| no addition of compound to be tested | 1.07 ± 0.14 (n=9) |
| 0.01 µM of compound to be tested | 1.56 ± 0.17 (n=9) |

### 〈Experiment 4〉

| | mean value ± standard error (unit: standardized area) |
|---|---|
| no addition of compound to be tested | 1.00 ± 0.13 (n=5) |
| 0.01 µM of compound to be tested | 1.31 ± 0.15 (n=6) |

In all of the above experiments, the function of promoting the outgrowth of a neurite was detected (in Experiments 1 and 2, significant level: p < 0.05), thereby making it clear that the compound has the function of promoting the outgrowth of a neurite.

Compared with the effect of CNTF of Reference Example 3, a neurite outgrowth promoting activity was detected even in the case of 1-day culture after addition. Therefore, it is considered that it is fairly possible that the effect of the compound is directly obtained by the promotion of the outgrowth of a neurite.

### Example 2

### evaluation of activity of promoting the outgrowth of neurite (2)

### compound to be tested: (16S)-15-deoxy-16-hydroxy-16-methyl-9(O)-methano-Δ^{6(9α)}-prostaglandin I₁

The evaluation method of Reference Example 2 (sample is the same as in Experiment 2 of Example 1) is used. The results are shown below.

| | means value ± standard error (unit: number of neurites) |
|---|---|
| no addition of compound to be tested | 40.3 ± 5.5 |
| 0.01 µM of compound to be tested | 87.2 ± 9.4 |

The neurite outgrowth promoting activity of the material to be tested was detected in this measurement method (significant level: p < 0.05).

The neurite outgrowth measuring method of Reference Example 2 used in this Example is a method for measuring the extension rate of a neurite more accurately than the measurement method of Reference Example 1. Therefore, the results of this example show more accurately that the compound has the function of promoting the outgrowth of a neurite.

### Example 3

### evaluation of activity of promoting outgrowth of neurite (3)

### compound to be tested: 9(O)-methano-Δ^{6(9α)}-prostaglandin I₁ (isocarbacyclin derivatives)

The same evaluation method as in Reference Example 1 was used. The closest distance between the slice of the dentate gyrus of hippocampus and the slice of the entorhinal cortex was set to 1 mm, the exchange of the culture medium was carried out one more time on the fourth day of culture with the same concentration of the compound by adding the material to be tested, and culture was carried out for 7 days in total.

The results are shown below. The area occupied by neurites extending over the collagen layer was calculated when that of the control (1.16 mm²) was 1.

| | mean value ± standard error (unit: standardized area) |
|---|---|
| no addition of compound to be tested | 1.00 ± 0.36 (n=5) |
| 0.01 µM of compound to be tested | 3.69 ± 0.29 (n=5) |

It has been made clear that the compound has the function of promoting the outgrowth of a neurite (significant level: p < 0.05).

### Example 4

### evaluation of activity of promoting the outgrowth of neurite (4)

### compound to be tested: methyl ester of 9(O)-methano-Δ^{6(9α)}-prostaglandin I₁ (isocarbacycline)

An experiment was carried out in the same manner as in Example 3.

The results are shown below. The area occupied by neurites outgrowing over the collagen layer was calculated when that of the control (1.16 mm²) was 1.

| | mean value ± standard error (unit: standardized area) |
|---|---|
| no addition of compound to be tested | 1.00 ± 0.36 (n=5) |
| 0.1 µM of compound to be tested | 3.97 ± 1.21 (n=5) |

It has been made clear that the compound has the function of promoting the outgrowth of a neurite (significant level: p < 0.05).

### Example 5

### measurement of learning and memory impairment ameliorating effect (1)

### compound to be tested: (16S)-15-deoxy-16-hydroxy-16-methyl-9(O)-methano-Δ^{6(9α)}-prostaglandin I₁

The leaning and memory impairment ameliorating effect of the compound was measured in accordance with the evaluation methods of Reference Examples 3 and 5. The compound to be tested was dissolved in phosphate buffered saline and injected with an osmotic pump simultaneous with the injection of β-amyloid protein. In Example 5, β-amyloid protein (40-1) or β-amyloid protein (1-40) was injected into the right cerebral ventricle of the rat and the phosphate buffered saline or a solution of the compound to be tested was injected into the left cerebral ventricle.

### (Experiment 1)

| | mean value of time required for rat to arrive at the platform ± standard error (unit: second) |
|---|---|
| a group of rats administered with 300 pmol/day of β-amyloid protein (40-1) | 9.7 ± 1.3 (n=10) |
| a group of rats administered with 300 pmol/day of β-amyloid protein (1-40) | 14.6 ± 3.0 (n=12) |
| a group of rats administered with 300 pmol/day of β-amyloid protein (1-40) and 120 pmol/day of compound to be tested | 12.7 ± 2.0 (n=10) |
| a group of rats administered with 300 pmol/day of β-amyloid protein (1-40) and 12 pmol/day of compound to be tested | 11.9 ± 2.0 (n=10) |

### (Experiment 2)

| | mean value of time required for rat to arrive at the platform ± standard error (unit: second) |
|---|---|
| a group of rats administered with 300 pmol/day of β-amyloid protein (40-1) | 9.0 ± 1.0 (n=5) |
| a group of rats administered with 300 pmol/day of β-amyloid protein (1-40) | 18.0 ± 3.3 (n=5) |
| a group of rats administered with 300 pmol/day of β-amyloid protein (1-40) and 1.2 pmol/day of compound to be tested | 11.1 ± 2.5 (n=5) |
| a group of rats administered with 300 pmol/day of β-amyloid protein (1-40) and 0.12 pmol/day of compound to be tested | 9.8 ± 1.5 (n=5) |

That is, in the above experiments, the compound exhibited a learning and memory impairment ameliorating effect.

### Example 6

### measurement of learning and memory impairment ameliorating effect (2)

### compound to be tested: (16S)-15-deoxy-16-hydroxy-16-methyl-9(O)-methano-Δ^{6(9α)}-prostaglandin I₁

The learning and memory impairment ameliorating effect of the compound was measured with the evaluation methods of Reference Examples 3 and 5. The compound to be tested was dissolved in a solution of β-amyloid protein and injected with an osmotic pump simultaneous with the injection of β-amyloid protein.

| | mean value of time required for rat to arrive at the platform ± standard error (unit: second) |
|---|---|
| a group of rats administered with 300 pmol/day of β-amyloid protein (40-1) | 8.1 ± 0.9 (n=10) |
| a group of rats administered with 300 pmol/day of β-amyloid protein (1-42) | 11.1 ± 1.5 (n=11) |
| a group of rats administered with 300 pmol/day of β-amyloid protein (1-42) and 0.12 pmol/day of compound to be tested | 7.0 ± 0.7 (n=10) |

That is, the compound exhibited a leaning and memory impairment ameliorating effect (significant level: p < 0.05).

### Example 7

### measurement of learning and memory impairment ameliorating effect (3)

### compound to be tested: (16S)-15-deoxy-16-hydroxy-16-methyl-9(O)-methano-Δ^{6(9α)}-prostaglandin I₁

The learning and memory impairment ameliorating effect of the compound was measured with the evaluation method of Reference Examples 4 and 5. The compound to be tested was dissolved in a solution of β-amyloid protein and injected with an osmotic pump simultaneous with the injection β-amyloid protein.

### (Experiment 1)

| | mean value of time required for rat to move to dark room on retention trial ± standard error (unit: second) |
|---|---|
| a group of rats administered with 300 pmol/day of β-amyloid protein (40-1) | 266.3 ± 23.6 (n=6) |
| a group of rats administered with 300 pmol/day of β-amyloid protein (1-42) | 147.5 ± 54.8 (n=4) |
| a group of rats administered with 300 pmol/day of β-amyloid protein (1-42) and 12 fmol/day of compound to be tested | 300.0 ± 0.0 (n=4) |
| a group of rats administered with 300 pmol/day of β-amyloid protein (1-42) and 120 fmol/day of compound to be tested | 262.1 ± 37.9 (n=7) |

### (Experiment 2)

| | mean value of time required for rat to move to dark room on retention trial ± standard error (unit: second) |
|---|---|
| a group of rats administered with 300 pmol/day of β-amyloid protein (40-1) | 255.6 ± 29.6 (n=8) |
| a group of rats administered with 300 pmol/day of β-amyloid protein (1-42) | 147.6 ± 38.2 (n=8) |
| a group of rats administered with 300 pmol/day of β-amyloid protein (1-42) and 1.2 fmol/day of compound to be tested | 188.1 ± 52.9 (n=7) |
| a group of rats administered with 300 pmol/day of β-amyloid protein (1-42) and 12 fmol/day of compound to be tested | 244.5 ± 37.6 (n=8) |
| a group of rats administered with 300 pmol/day of β-amyloid protein (1-42) and 120 fmol/day of compound to be tested | 244.0 ± 35.8 (n=8) |

### (Experiment 3)

| | mean value of time required for rat to move to dark room on retention trial ± standard error (unit: second) |
|---|---|
| a group of rats administered with 300 pmol/day of β-amyloid protein (40-1) | 277.3 ± 22.7 (n=3) |
| a group of rats administered with 300 pmol/day of β-amyloid protein (1-42) | 79.5 ± 18.0 (n=4) |
| a group of rats administered with 300 pmol/day of β-amyloid protein (1-42) and 1.2 fmol/day of compound to be tested | 186.0 ± 41.2 (n=4) |
| a group of rats administered with 300 pmol/day of β-amyloid protein (1-42) and 120 fmol/day of compound to be tested | 241.0 ± 59.0 (n=4) |

### (Experiment 4)

| | mean value of time required for rat to move to dark room on retention trial ± standard error (unit: second) |
|---|---|
| a group of rats administered with 300 pmol/day of β-amyloid protein (40-1) | 232.1 ± 44.2 (n=7) |
| a group of rats administered with 300 pmol/day of β-amyloid protein (1-42) | 171.0 ± 42.6 (n=9) |
| a group of rats administered with 300 pmol/day of β-amyloid protein (1-42) and 0.12 fmol/day of compound to be tested | 195.1 ± 49.9 (n=7) |
| a group of rats administered with 300 pmol/day of β-amyloid protein (1-42) and 12 fmol/day of compound to be tested | 247.6 ± 36.7 (n=8) |

That is, in the above experiments, the compound exhibited a dose-dependent learning and memory impairment ameliorating effect.

### Effect of the Invention

The drug of the present invention can be a drug for degenerative diseases of the brain or nerve accompanied by dementia such as dementia of Alzheimer type, Parkinson disease, multiple sclerosis, lateral sclerosis and diabetic neuropathy, or motor disorders. It can also be a drug for improving the functional disorders of the brain caused by cerebral infarction and cerebral apoplexy. Further, it can be a drug for nerve injuries caused by external wounds.

## Claims

1. A drug for a nervous disorder comprising a prostaglandin represented by the following formula (I): 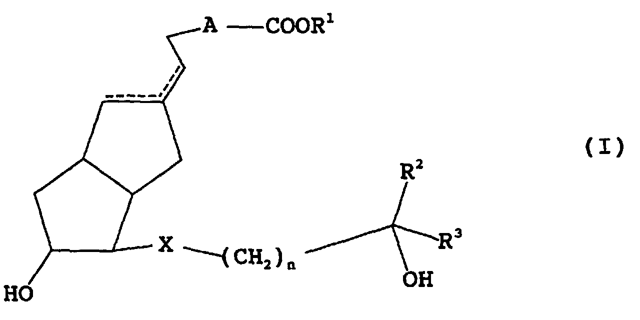 wherein R¹ is a hydrogen atom, C₁∼C₁₀ alkyl group or 1-equivalent cation, R² is a hydrogen atom, methyl group or vinyl group, R³ is a linear or branched C₃∼C₁₀ alkyl group, linear or branched C₃∼C₁₀ alkenyl group, linear or branched C₃∼C₁₀ alkynyl group, C₃∼C₁₀ cycloalkyl group which may be substituted, phenyl group which may be substituted, phenoxy group which may be substituted, or linear or branched C₁∼C₆ alkyl group which is substituted with a phenyl group which may be substituted, phenoxy group which may be substituted or C₃∼C₁₀ cycloalkyl group which may be substituted, n is 0 or 1, A is -CH₂-CH₂-, -CH=CH- or -O-CH₂-, X is -CH₂-CH₂-, -CH(NH₂)-CH₂-, -CH=CH-, -C(NH₂)=CH- or -C≡C-, and the bond 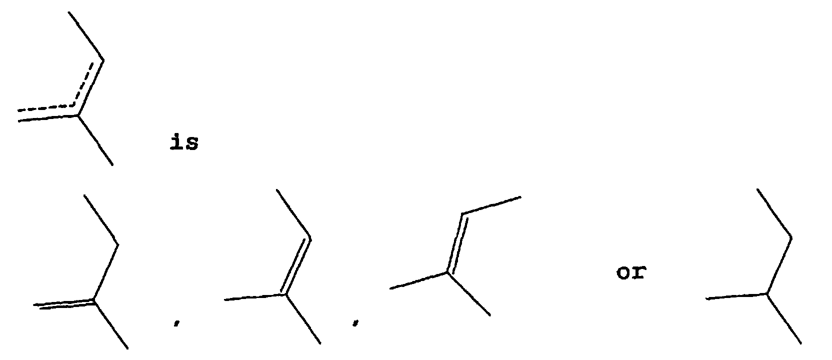 with the proviso that when A is -O-CH₂- and X is -CH(NH₂)-CH₂-or -C(NH₂)=CH-, the bonding direction may be opposite, and/or an optical isomer thereof as an active ingredient(s).

2. A drug for a nervous disorder comprising a prostaglandin represented by the following formula (II): 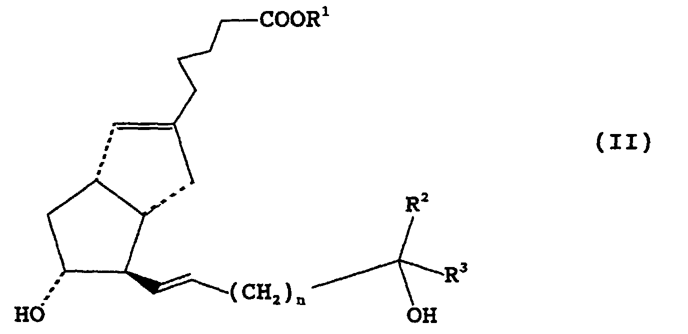 wherein R¹, R², R³ and n are as defined in the above formula (I),
and/or an optical isomer thereof as an active ingredient(s).

3. The drug for a nervous disorder of claim 2, wherein R¹ is a hydrogen atom or methyl group in the above formula (II).

4. The drug for a nervous disorder of claim 2, wherein R¹ is a hydrogen atom or methyl group, n is 0, R² is a hydrogen atom, and R³ is a pentyl group in the above formula (II).

5. The drug for a nervous disorder of claim 2, wherein R¹ is a hydrogen atom or methyl group, n is 1, R² is a methyl group, and R³ is a butyl group in the above formula (II).

6. A drug for a nervous disorder comprising a prostaglandin represented by the following formula (III): 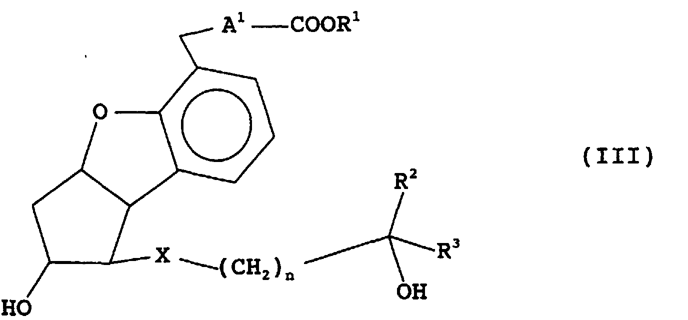 wherein R¹, R², R³, X and n are as defined in the above formula (I), and A¹ is -CH₂-CH₂- or -CH=CH-, with the proviso that when X is -CH(NH₂)-CH₂- or -C(NH₂)=CH-, the bonding direction may be opposite, and/or an optical isomer thereof as an active ingredient(s).

7. A drug for a nervous disorder comprising a prostaglandin represented by the following formula (IV): 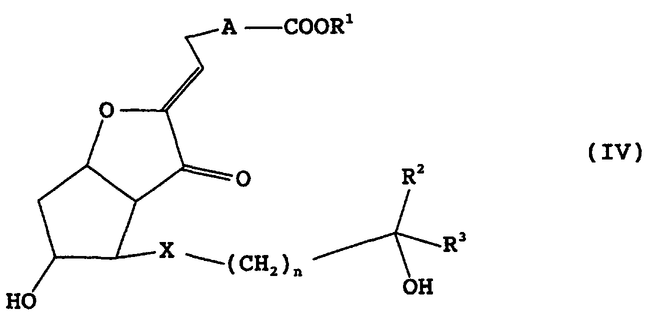 wherein R¹, R², R³, A, X and n are as defined in the above formula (I) with the same proviso as in the above formula (I), and/or an optical isomer thereof as an active ingredient(s).

8. A drug for a nervous disorder selected from the group consisting of dementia of Alzheimer type, Parkinson disease, multiple sclerosis, lateral sclerosis, diabetic peripheral neuropathy, learning and memory impairments, nerve injuries and the functional disorders of the brain caused by cerebral infarction and cerebral apoplexy, which comprises a prostaglandin represented by the above formula (I), (III) or (IV) and/or an optical isomer thereof as an active ingredient(s).

9. Use of a prostaglandin represented by the above formula (I), (III) or (IV) and/or an optical isomer thereof as an active ingredient(s) for the treatment of a nervous disorder.

10. A method for therapy of a nervous disorder which comprises administering a prostaglandin represented by the above formula (I), (III) or (IV) and/or an optical isomer thereof at a therapeutically effective amount of the nervous disorder to a patient having the nervous disorder.
